# EUROPEAN PATENT APPLICATION

(11) **EP 2 783 628 A1**
(43) Date of publication of application: **01.10.2014**
(21) Application number: 14000050.6
(22) Date of filing: 08.01.2014
(51) Int. Cl.: A61B 5/044, A61B 5/04, A61B 5/0402, A61B 5/00

(54) **Biopotential measurement device**

(30) Priority: 27.03.2013 JP 2013065703
(71) Applicant: Tanita Corporation, Tokyo 174-8630 (JP)
(72) Inventor: Ishige, Tadaaki, Tokyo, 174-8630 (JP)
(74) Representative: Müller-Boré & Partner Patentanwälte PartG mbB

(57) **Abstract**

A biopotential measurement device (10) includes a measurement unit (14) and a display unit (16). The measurement unit (14) includes electrodes on a measurement surface. The electrodes detect the electric potential, at detection positions, of a living organism facing the measurement surface. The detection positions are determined within the measurement area. The display unit (16) is positionable on a surface opposite the measurement surface. The display unit (16) displays the electric potential detected at each detection position at positions within a display area. The positions within the display area correspond to the detection positions within the measurement area.

## Description

### TECHNICAL FIELD

The present invention relates to a biopotential measurement device for measuring the electric potential of a living organism at a plurality of positions.

### BACKGROUND ART

The electric potential distribution over the body surface of a living organism can aid a user, such as a doctor, in understanding the condition of the living organism. For example, it has been proposed to measure the electrical potential of the body surface in the chest region using a plurality of electrodes arrayed in a two-dimensional shape and to create an electric potential map (see JP4272703 (PTL 1)).

### CITATION LIST

### Patent Literature

PTL 1: JP4272703

### SUMMARY OF INVENTION

In order to understand the condition of the living organism accurately, it is necessary to recognize accurately where in the living organism the visually observed electric potential was detected. With the electric potential map that is rendered by the body surface electrocardiograph disclosed in PTL 1, however, an accurate understanding of the location on the body surface to which a particular electric potential in the map corresponds depends on the skill of the user.

The present invention has been conceived in light of the above perspective, and it is an object thereof to provide a biopotential measurement device that allows for easy recognition of the detection position corresponding to the detected electric potential.

In order to resolve the above-described problems, a biopotential measurement device according to a first aspect comprises a measurement unit including, on a measurement surface, at least one electrode detecting electric potential of a living organism at at least one detection position determined within a measurement area, the living organism facing the measurement surface; and a display unit positionable on a surface opposite the measurement surface and configured to display the electric potential detected at the at least one detection position within the measurement area at at least one display position, within a display area of the display unit, corresponding to the at least one detection position.

In a biopotential measurement device according to a second aspect, the display unit preferably displays the electric potential detected at the at least one detection position with an isoelectric line.

In a biopotential measurement device according to a third aspect, the display unit is preferably detachable from the measurement unit.

A biopotential measurement device according to a fourth aspect preferably further comprises a control unit configured to determine whether the electrode is in contact with the living organism.

A biopotential measurement device according to a fifth aspect preferably further comprises a drive unit configured to displace the measurement unit towards the living organism, the at least one electrode preferably comprises a plurality of electrodes, and the drive unit preferably displaces the measurement unit towards the living organism until the control unit determines that all of the electrodes are in contact with the living organism.

In a biopotential measurement device according to a sixth aspect, the at least one detection position preferably comprises a plurality of detection positions, the at least one electrode preferably comprises a plurality of electrodes, and the measurement unit preferably includes the electrodes in one-to-one correspondence with the detection positions.

In a biopotential measurement device according to a seventh aspect, the measurement unit is preferably displaceable to a plurality of displacement positions determined within the measurement area, the at least one detection position preferably comprises a plurality of detection positions, and the electric potential of the living organism is preferably detectable at all of the detection positions by detecting the electric potential of the living organism at all of the displacement positions.

According to the present invention, the detected electric potential can be displayed so that the corresponding position at which the electric potential was detected can be easily recognized.

### BRIEF DESCRIPTION OF DRAWINGS

The present invention will be further described below with reference to the accompanying drawings, wherein:
FIG. 1 is an external view of a biopotential measurement device according to an embodiment of the present invention;
FIG. 2 illustrates a method of measuring the electric potential of a living organism using the biopotential measurement device in FIG. 1;
FIG. 3 is a functional block diagram schematically illustrating the internal structure of the biopotential measurement device;
FIG. 4 is an external view with the lower surface of the main unit in FIG. 1 facing upwards;
FIG. 5 illustrates a biopotential map displayed within the display surface in FIG. 1;
FIG. 6 illustrates a waveform chart displayed within the display surface in FIG. 1;
FIG. 7 illustrates a waveform chart displayed within the display surface when particular electrodes are selected;
FIG. 8 is a flowchart showing processing executed by the control unit for creation of a diagram;
FIG. 9 is an external view of a modification to the biopotential measurement device; and
FIG. 10 is a bottom view of the main unit in FIG. 9.

### DESCRIPTION OF EMBODIMENTS

The following describes an embodiment of the present invention with reference to the drawings.

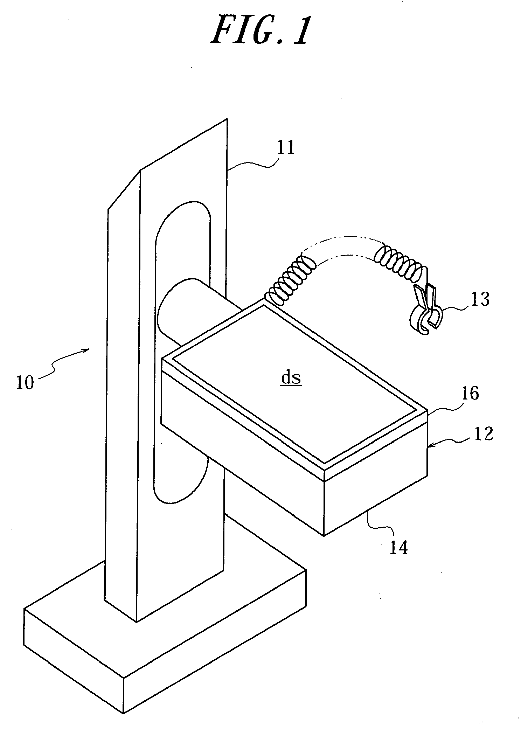

FIG. 1 is an external view of a biopotential measurement device according to an embodiment of the present invention.

As illustrated in FIG. 1, the biopotential measurement device 10 is, for example, an electrocardiograph and includes a supporting column 11, a main unit 12, and a reference electrode 13.

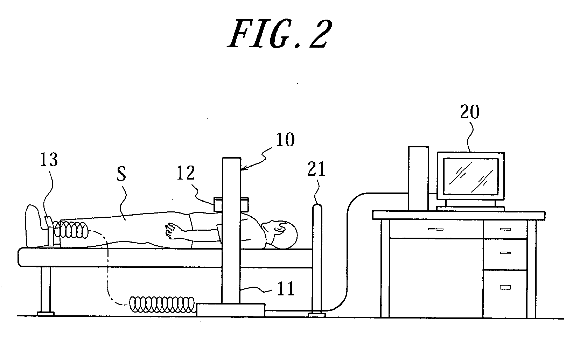

The supporting column 11 includes a displacement mechanism and supports the main unit 12 displaceably in the longitudinal direction of the supporting column 11. As illustrated in FIG. 2, the main unit 12 detects the electric potential on the body surface while in contact with a subject S and displays the detected electric potential. The reference electrode 13 applies a reference potential for the body surface electric potential to the subject S.

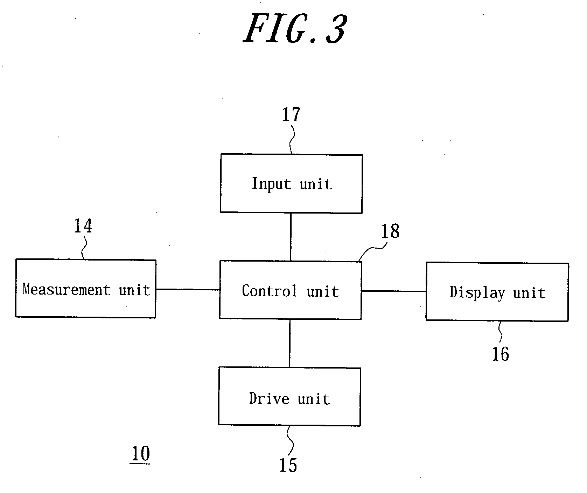

Next, details on the internal structure of the biopotential measurement device 10 are provided. As illustrated in FIG. 3, the biopotential measurement device 10 includes a measurement unit 14, a drive unit 15, a display unit 16, an input unit 17, a control unit 18, and the like.

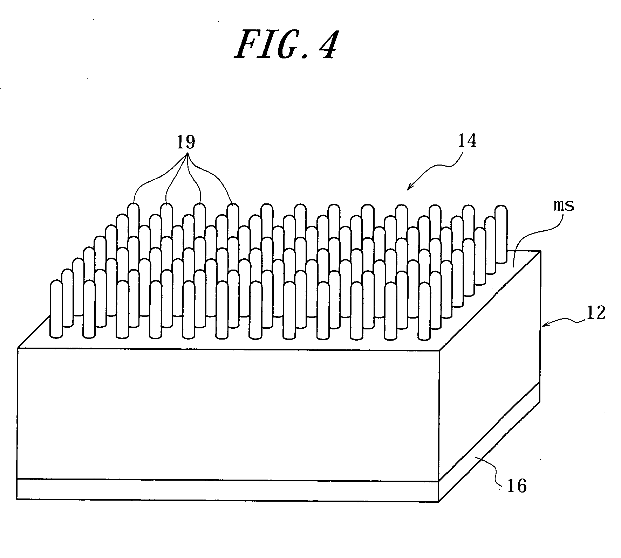

The measurement unit 14 is provided in the main unit 12. As illustrated in FIG. 1, the main unit 12 is, for example, a box shape, and the measurement unit 14 is provided on one surface thereof (in FIGS. 1 and 2, the lower surface). The surface of the main unit 12 shared by the measurement unit 14 is designated a measurement surface ms (see FIG. 4). The positions at which the electric potential of the living organism facing the measurement surface ms is detected are determined in advance within the measurement area as detection positions. The measurement area is an area within the measurement surface ms having the same size as a display surface of the display unit 16. As illustrated in FIG. 4, an electrode 19 that detects the electric potential of the contacting living organism is provided at each detection position. Note that in the present embodiment, 8 × 12, for a total of 96, electrodes 19 are provided, yet the number of electrodes 19 is not limited to 96. Each electrode 19 has a coiled spring or the like and can contact the living organism by expansion or contraction of the coiled springs, including along an uneven surface of the living organism.

The drive unit 15 is, for example, incorporated into the displacement mechanism of the supporting column 11. The drive unit 15 is a motor, for example, and displaces the main unit 12 in the longitudinal direction of the supporting column 11 based on an instruction from the control unit 18.

The display unit 16 is provided on the surface of the main unit 12 opposite the measurement surface ms. The display unit 16 has, for example, a structure integral with the main unit 12 and has a display surface ds (see FIG. 1) overlapping the measurement surface ms. The display unit 16 may, for example, be detachable from the main unit 12, with the display surface ds overlapping the measurement surface ms when the display unit 16 is placed on the main unit 12. As described below, the display unit 16 displays a diagram created by the control unit 18 illustrating the electric potential detected by each electrode 19 within the display surface ds.

The input unit 17 (see FIG. 3) is, for example, provided in the supporting column 11. Alternatively, a touch panel may be adopted in the display unit 16 and caused to function as the input unit 17. The input unit 17 includes a plurality of buttons or the like and detects a variety of input from the user to the biopotential measurement device 10.

The control unit 18 is, for example, provided in the main unit 12. The control unit 18 executes a variety of processing to cause the biopotential measurement device 10 to function and controls the operations of each unit in the biopotential measurement device 10. For example, the control unit 18 determines whether each electrode 19 is in contact with the living organism based on the electric potential detected by each electrode 19 and acquired from the measurement unit 14. Based on input detected by the input unit 17 and on whether the electrodes 19 are in contact with the living organism, the control unit 18 also controls the drive unit 15 to displace the main unit 12 in the longitudinal direction of the supporting column 11. Furthermore, the control unit 18 creates a diagram such as a biopotential map or waveform chart illustrating the electric potential detected by the electrodes 19 and also causes the display unit 16 to display the created diagram within a display area designated as an area for displaying electric potential. The display area is an area displaying the created diagram within the display surface ds and corresponds to either the entire display surface ds or a partial area thereof. The control unit 18 can also transmit a variety of acquired data to an external device 20 such as a personal computer (see FIG. 2).

Next, the control of the drive unit 15 executed by the control unit 18 is described in detail. In order to detect the surface electric potential of the living organism, the electrodes 19 need to be brought into contact with the living organism. Therefore, as illustrated in FIG. 2, the biopotential measurement device 10 is configured so that the main unit 12 is displaced downwards from above to allow the electrodes 19 to contact the chest region of a subject S lying face-up on a bed 21 or the like. For example, through input into the input unit 17 to raise the main unit 12, the drive unit 15 can be caused to raise the main unit 12 and bring the electrodes 19 into contact with the subject S. Furthermore, through input into the input unit 17 for automatic position adjustment of the main unit 12, the control unit 18 can bring the electrodes 19 into contact with the subject S by controlling the drive unit 15 to displace the main unit 12 towards the subject S until determining that all of the electrodes 19 are in contact with the subject S.

Next, the diagram created by the control unit 18 is described. The control unit 18 can create a variety of diagrams to illustrate electric potential. For example, the control unit 18 can create a biopotential map, a waveform chart, or the like.

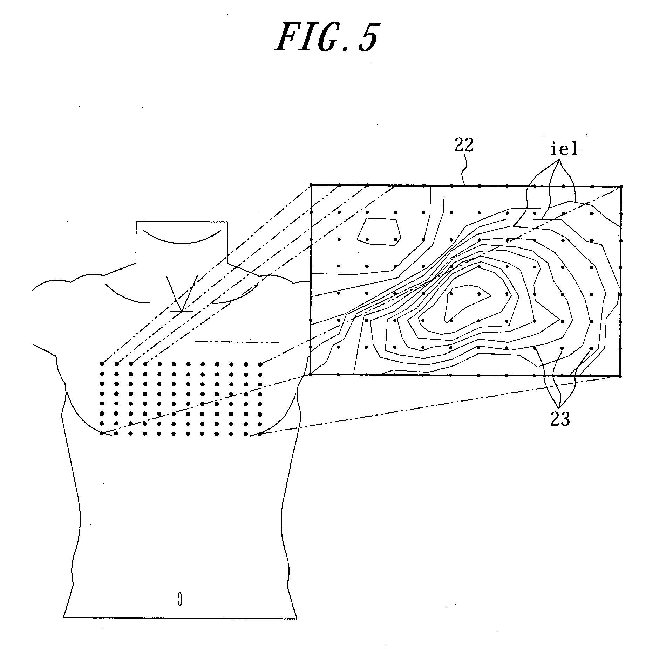

As illustrated in FIG. 5, a biopotential map is a distribution map that uses isoelectric lines iel to show the magnitude, at each position, of the electric potential of the living organism facing the measurement surface ms. Based on the value of the electric potential of each electrode 19 and the coordinates of each electrode 19 acquired from the measurement unit 14, the control unit 18 estimates the electric potential at each position between the electrodes 19 by interpolation. Based on the estimated electric potentials, the control unit 18 renders isoelectric lines iel. The outline of the biopotential map 22 is substantially equivalent in shape to the measurement area, and the electric potential at each position within the measurement area is displayed at a position that is relatively the same within the biopotential map 22. The size of the biopotential map 22 can be adjusted, yet when the biopotential map 22 is the same size as the measurement area, the electric potential at a given position of the measurement area is displayed at the same position of the display surface ds, which is on the opposite side of the measurement surface ms. Furthermore, each detection position is displayed in the biopotential map 22 with, for example, a black dot 23.

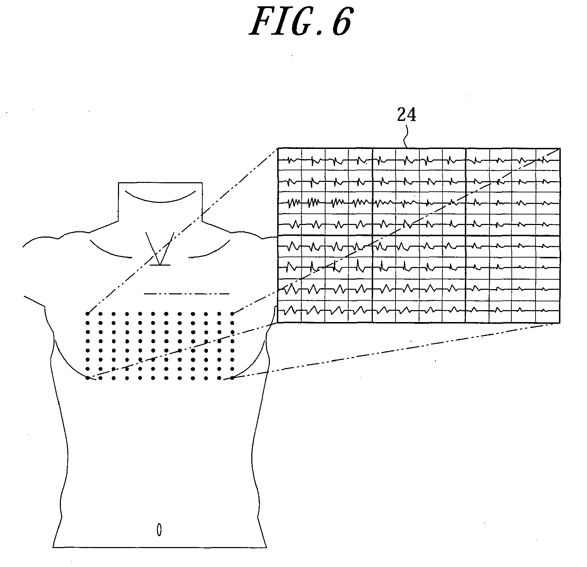

A waveform chart is, as illustrated in FIG. 6, a graph displaying a waveform of the variation in electric potential over time at every position corresponding to the electrodes 19. In other words, the electric potentials detected by the electrodes 19 provided at 96 detection positions in the present embodiment are displayed at positions that are relatively the same within the waveform chart 24.

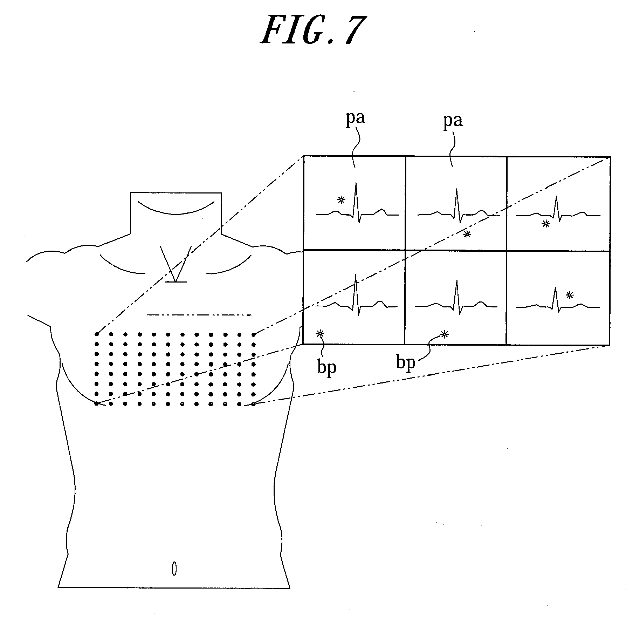

In the waveform chart 24, the electric potentials detected by all of the electrodes 19 are displayed, yet by input into the input unit 17, particular detection positions can be selected, and a waveform chart of the electric potential detected by the electrode 19 at each selected detection position can be created. Either one or a plurality of detection positions may be selected, and for example, as illustrated in FIG. 7, the display area can be divided into small areas pa, and each of the electric potentials detected by the electrodes 19 at the selected detection positions can be displayed in a corresponding one of the small areas pa. When the detection position is selected in a small area pa, the selected detection position can also be caused to blink (see reference sign "bp").

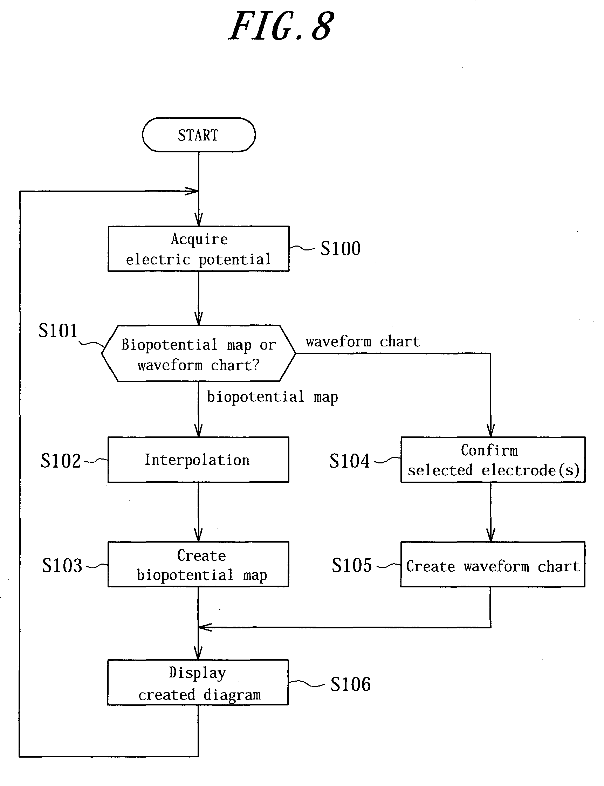

Next, the processing executed by the control unit 18 for creation of a diagram displaying electric potential is described with reference to the flowchart in FIG. 8. The creation processing begins upon detection in the input unit 17 of input providing an instruction for display of a diagram while the electrodes 19 are in contact with the subject S. The creation processing is repeatedly executed until detection in the input unit 17 of input providing an instruction for execution of an operation other than display of a diagram.

In step S100, the control unit 18 acquires the electric potential detected for each electrode 19. The control unit 18 stores the acquired electric potential for each corresponding electrode 19 in a work memory, such as an SDRAM, of the control unit 18. Upon acquisition of the electric potentials, processing proceeds to step S101.

In step S101, the control unit 18 determines whether creation of a biopotential map 22 or a waveform chart 24 has been selected. Through input to the input unit 17, the user can select the diagram to be created in advance or afterwards. When the biopotential map 22 has been selected, processing proceeds to step S102. When the waveform chart 24 has been selected, processing proceeds to step S104.

In step S102, the control unit 18 calculates the electric potentials between electrodes 19 by interpolation based on the electric potentials acquired in step S100. Upon calculation of the electric potentials between electrodes 19, processing proceeds to step S103.

In step S103, the control unit 18 creates a biopotential map 22 based on the electric potentials calculated in step S102. Once the biopotential map 22 has been created, processing proceeds to step S106.

In step S104, to which processing proceeds when the waveform chart 24 has been selected in step S101, the control unit 18 confirms the selected electrode(s) 19. Selection of the electrode(s) 19 may be made by input to the input unit 17 before or after creation of the waveform chart 24. In the case of creation before selection of the electrode(s) 19, the control unit 18 considers all of the electrodes 19 to have been selected. Upon confirmation of the selected electrode(s) 19, processing proceeds to step S105.

In step S105, the control unit 18 creates a waveform chart 24 that includes an electric potential waveform for each electrode 19 confirmed as selected in step S104. To create the waveform, the control unit 18 uses the electric potentials acquired and stored in the work memory in step S100.

In step S106, the control unit 18 displays either the biopotential map 22 created in step S103 or the waveform chart 24 created in step S105 on the display unit 16. After displaying the created diagram, processing returns to step S100.

According to the biopotential measurement device of the present embodiment with the above structure, the display unit 16 is provided on the surface opposite the measurement surface ms, and in this state, the electric potential detected at each detection position can be displayed at a corresponding position in the display area. Accordingly, the user can easily discern the detection position of each displayed electric potential, and hence the biopotential measurement device 10 can contribute to a rapid and clear understanding and diagnosis of a living organism's condition.

Furthermore, according to the biopotential measurement device of the present embodiment, the control unit 18 can determine whether the electrodes 19 are in contact with the living organism and can therefore spare the user the task of visual confirmation. User-friendliness can thus be improved. Moreover, according to the biopotential measurement device of the present embodiment, the main unit 12 is displaced until all of the electrodes 19 are in contact with the living organism, thereby eliminating the need for fine control over displacement of the main unit 12 and further improving user-friendliness.

The present invention has been described based on the drawings and an embodiment, yet it should be noted that a person of ordinary skill in the art can easily make a variety of modifications and adjustments based on the present disclosure. Accordingly, these modifications and adjustments should be understood as being included within the scope of the present invention.

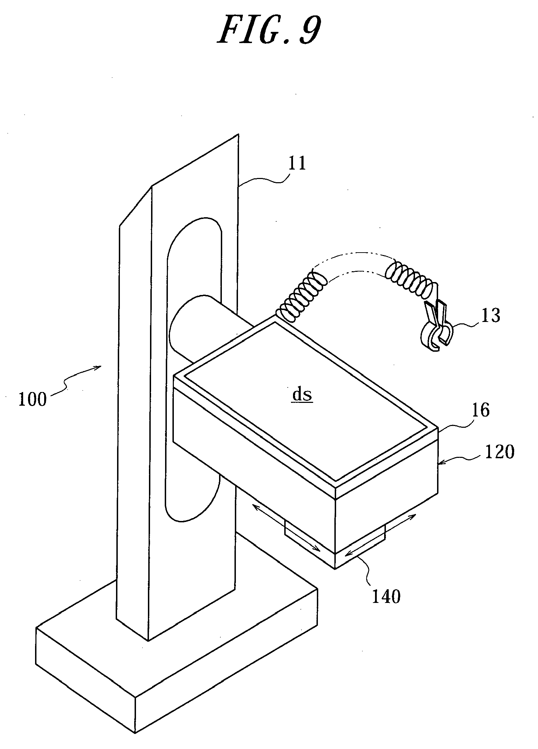

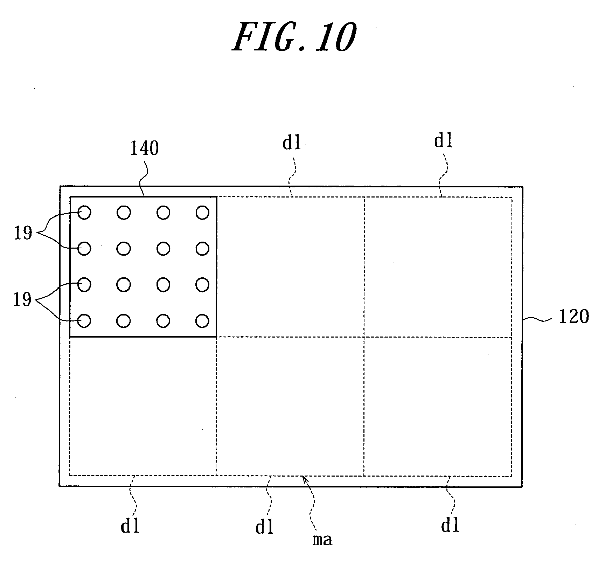

For example, in the above embodiment, one of the electrodes 19 is provided at each detection position in the measurement area, yet an electrode 19 need not be provided at every detection position. For example, as illustrated in FIG. 9, in a biopotential measurement device 100, a measurement unit 140 may be displaceable along the bottom surface of a main unit 120. As illustrated in FIG. 10, by consecutively displacing the measurement unit 140 to displacement positions dl determined within the measurement area ma and detecting the electric potential at each displacement position dl, the electric potential of the living organism at every detection position within the measurement area ma can be detected.

The biopotential measurement device of the above embodiment is an electrocardiograph, yet the biopotential measurement device of the present invention may be adopted in any device that detects electric potential on the surface of a living organism. Such a device may, for example, be a device that detects an electromyogram, an electrogastrogram, an electroencephalogram, or the like.

### REFERENCE SIGNS LIST

- 10, 100:: Biopotential measurement device
- 11:: Supporting column
- 12, 120:: Main unit
- 13:: Reference electrode
- 14, 140:: Measurement unit
- 15:: Drive unit
- 16:: Display unit
- 17:: Input unit
- 18:: Control unit
- 19:: Electrode
- 20:: External device
- 21:: Bed
- 22:: Biopotential map
- 23:: Detection position
- 24:: Waveform chart
- dl:: Displacement position
- ds:: Display surface
- ma:: Measurement area
- ms:: Measurement surface
- pa:: Small area
- S:: Subject

## Claims

1. A biopotential measurement device comprising:
a measurement unit including, on a measurement surface, at least one electrode detecting electric potential of a living organism at at least one detection position determined within a measurement area, the living organism facing the measurement surface; and
a display unit positionable on a surface opposite the measurement surface and configured to display the electric potential detected at the at least one detection position within the measurement area at at least one display position, within a display area of the display unit, corresponding to the at least one detection position.

2. The biopotential measurement device according to claim 1, wherein the display unit displays the electric potential detected at the at least one detection position with an isoelectric line.

3. The biopotential measurement device according to claim 1 or 2, wherein the display unit is detachable from the measurement unit.

4. The biopotential measurement device according to any one of claims 1 through 3, further comprising a control unit configured to determine whether the at least one electrode is in contact with the living organism.

5. The biopotential measurement device according to claim 4, further comprising a drive unit configured to displace the measurement unit towards the living organism, wherein
the at least one electrode comprises a plurality of electrodes, and
the drive unit displaces the measurement unit towards the living organism until the control unit determines that all of the electrodes are in contact with the living organism.

6. The biopotential measurement device according to any one of claims 1 through 5, wherein
the at least one detection position comprises a plurality of detection positions,
the at least one electrode comprises a plurality of electrodes, and
the measurement unit includes the electrodes in one-to-one correspondence with the detection positions.

7. The biopotential measurement device according to any one of claims 1 through 5, wherein
the measurement unit is displaceable to a plurality of displacement positions determined within the measurement area,
the at least one detection position comprises a plurality of detection positions, and
the electric potential of the living organism is detectable at all of the detection positions by detecting the electric potential of the living organism at all of the displacement positions.
